# EUROPÄISCHE PATENTSCHRIFT

(11) **EP 0 912 486 B1**
(45) Veröffentlichungstag und Bekanntmachung des Hinweises auf die Patenterteilung: **25.10.2000**
(21) Anmeldenummer: 97929277.8
(22) Anmeldetag: 24.06.1997
(51) Int. Cl.: C07C 57/04, C07C 51/43

(54) **VERFAHREN ZUR REINIGUNG VON ROH-ACRYLSÄURE DURCH KRISTALLISATION**
PROCESS FOR PURIFYING CRUDE ACRYLIC ACID BY CRYSTALLIZATION
PROCEDE DE PURIFICATION D'ACIDE ACRYLIQUE BRUT PAR CRISTALLISATION

(30) Priorität: 10.07.1996 DE 19627679
(43) Veröffentlichungstag der Anmeldung: 06.05.1999
(73) Patentinhaber: BASF AKTIENGESELLSCHAFT, 67056 Ludwigshafen (DE)
(72) Erfinder: LEHNERT, Klaus, D-67112 Mutterstadt (DE); MÜLLER-ENGEL, Klaus, Joachim, D-76297 Stutensee (DE); NESTLER, Gerhard, D-67061 Ludwigshafen (DE); ECK, Bernd, D-68519 Viernheim (DE)
(86) Internationale Anmeldenummer: EP9703304
(87) Internationale Veröffentlichungsnummer: WO9801414

(56) Entgegenhaltungen:
- Keine einschlägigen Dokumente gefunden

## Beschreibung

Vorliegende Erfindung betrifft ein Verfahren zur Reinigung von Roh-Acrylsäure durch Kristallisation.

Acrylsäure, entweder für sich oder in Form ihrer Salze oder ihrer Ester, ist insbesondere zur Herstellung von Polymerisaten für die verschiedensten Anwendungsgebiete (z. B. Klebstoffe, Superabsorber, Bindemittel) von Bedeutung.

Unter anderem ist Acrylsäure durch katalytische Gasphasenoxidationen von Propan, Propen und/oder Acrolein erhältlich. Dabei werden diese Ausgangsgase, in der Regel mit inerten Gasen wie Stickstoff, CO₂ und/oder Wasserdampf verdünnt, im Gemisch mit Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgemisch umgewandelt. Durch Kondensation des Produktgemisches oder durch Aufnahme in ein geeignetes Absorptionsmittel (z. B. Wasser oder ein Gemisch aus 70 bis 75 Gew.-% Diphenylether und 25 bis 30 Gew.-% Diphenyl) kann eine Grundabtrennung der Acrylsäure aus dem Produktgasstrom erzielt werden (vgl. z. B. EP-A 297 445 und DE-PS 21 36 396).

Durch Entfernung des Absorptionsmittels (und gegebenenfalls zuvor erfolgter Desorption von eine geringe Absorptionsmittellöslichkeit aufweisenden Verunreinigungen durch Abstreifen, z. B. mit Luft) über extraktive und/oder destillative Trennverfahren (z. B. Entfernung der Absorptionsmittels Wasser durch Destillation, azeotope Destillation oder extraktive Abtrennung der Säure aus der wäßrigen Lösung und anschließende destillative Entfernung des Extraktionsmittels) und/oder nach Anwendung von sonstigen Trennschritten wird häufig eine Acrylsäure erhalten, die hier als Roh-Acrylsäure bezeichnet wird.

Diese Roh-Acrylsäure ist kein reines Produkt. Vielmehr enthält sie ein Spektrum verschiedener, für den gasphasenkatalytisch oxidativen Herstellungsweg typische, Verunreinigungen. Diese sind insbesondere Essigsäure, Propionsäure, Wasser und niedermolekulare Aldehyde wie Acrolein, Methacrolein, Propionaldehyd, n-Butyraldehyd, Benzaldehyd, Furfurale und Crotonaldehyd.

Ein weiterer typischer Bestandteil von Roh-Acrylsäure sind Polymerisationsinhibitoren. Diese werden im Verlauf der zur Herstellung von Roh-Acrylsäure angewandten Trennprozesse zugesetzt, wo sie eine mögliche radikalische Polymerisation der α, β-monoethylenisch ungesättigten Acrylsäure unterdrücken sollen, weshalb sie auch als Prozeßstabilisatoren bezeichnet werden. Eine herausragende Position unter den Acrylsäure-Prozeßstabilisatoren nimmt Dibenzo-1,4-thiazin, 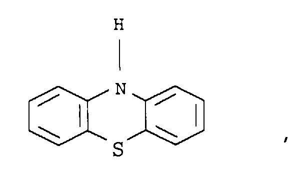 ein. Dibenzo-1,4-thiazin ist eine farblose, bei 180°C (Druck = 1 bar) schmelzende Substanz, die durch Erhitzen von Diphenylamin mit Schwefel erhältlich ist. Üblicherweise wird Dibenzo-1,4-thiazin entweder als alleiniger Acrylsäure-Prozeßstabilisator oder in Kombination mit anderen möglichen Acrylsäure-Prozeßstabilisatoren wie z. B. Hydrochinon angewendet, weshalb Dibenzo-1,4-thiazin ein charakteristischer Bestandteil der hier relevanten Roh-Acrylsäure ist.

Weitere unerwünschte Begleiter von in kondensierter Phase befindlicher Acrylsäure sind die durch Michael-Addition von Acrylsäure an sich selbst sowie an das sich dabei bildende Acrylsäure-Dimere entstehenden Acrylsäure-Oligomere (Michael-Addukte). Während diese Verbindungen in frisch erzeugter Roh-Acrylsäure normalerweise so gut wie nicht enthalten sind (üblicherweise beträgt ihr Gewichtsanteil < 0,01 Gew.-%), entstehen sie in selbiger bei längerem sich selbst überlassen. Aus statistischen Gründen ist dabei lediglich die Bildung von Diacrylsäure, 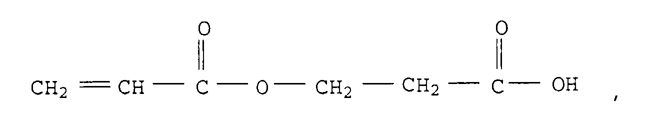 von Bedeutung, wohingegen die Bildung höherer Acrylsäure-Oligomere (Trimere, Tetramere etc.) im wesentlichen vernachlässigbar ist.

Bereits die Bildung der Diacrylsäure ist ein langsamer Prozeß. Überläßt man eine eine Reinheit von ≥ 99,5 Gew.-% aufweisende Acrylsäure bei 25°C, 1 bar sich selbst, so beträgt die Bildung an Diacrylsäure je Tag etwa 150 ppm, bezogen auf das Gewicht der Acrylsäure. Die Gesamtmenge an sonstigen in der Roh-Acrylsäure möglicherweise enthaltenen Nebenkomponenten beträgt auf das Gewicht der Roh-Acrylsäure bezogen in der Regel nicht mehr als 10 Gew.-%.

Unter Roh-Acrylsäure soll in dieser Schrift deshalb solche Acrylsäure verstanden werden, die, läßt man ihren Gehalt an Acrylsäure-Oligomeren ¹⁾ (Michael-Addukte) unberücksichtigt,
≥ 70 Gew.-% Acrylsäure,
≤ 20 Gew.-% Essigsäure,
≤ 5 Gew.-% Propionsäure
≤ 5 Gew.-% Wasser,
≤ 5 Gew.-% niedermolekulare Aldehyde und
≥ 0,80 Gew.-% Dibenzo-1,4-thiazin
enthält; d. h., die Angaben in Gew.-% sind auf das Gewicht der Roh-Acrylsäure abzüglich ihres Gehaltes an Acrylsäure-Oligomeren bezogen.
¹⁾ Acrylsäure-Oligomere meint in dieser Schrift stets die entsprechenden Michael-Addukte und nicht durch radikalische Polymerisation entstehende Acrylsäureoligomere, da die Bildung letzterer durch das Beisein von Polymerisationsinhibitoren im wesentlichen unterdrückt wird.

Insbesondere soll in dieser Schrift unter Roh-Acrylsäure solche Acrylsäure verstanden werden, die, läßt man ihren Gehalt an Acrylsäure-Oligomeren unberücksichtigt,
≥ 80 Gew.-% Acrylsäure,
≤ 15 Gew.-% Essigsäure,
≤ 5 Gew.-% Propionsäure,
≤ 5 Gew.-% Wasser,
≤ 5 Gew.-% niedermolekulare Aldehyde und
≥ 0,80 Gew.-% Dibenzo-1,4-thiazin
enthält.

Damit umfaßt der hier verwendete Begriff Roh-Acrylsäure auch solche Acrylsäure, die, läßt man ihren Gehalt an Acrylsäure-Olgiomeren unberücksichtigt,
≥ 90 Gew.-% Acrylsäure,
≤ 5 Gew.-% Essigsäure,
≤ 2 Gew.-% Propionsäure,
≤ 2 Gew.-% Wasser,
≤ 2 Gew.-% niedermolekulare Aldehyde und
≥ 0,80 Gew.-% Dibenzo-1,4-thiazin
enthält.

Von den in den vorgenannten Roh-Acrylsäuren neben Acrylsäure enthaltenen Bestandteilen erweisen sich die meisten im Rahmen einer Acrylsäureverwendung als nachteilig.

Würde eine solche Roh-Acrylsäure beispielsweise zur Herstellung von Estern aus C₁- bis C₈-Alkanolen und Acrylsäure verwendet, würden in Nebenreaktionen auch die entsprechenden Essigsäure- und Propionsäureester gebildet, was die Ausbeute an gewünschtem Acrylsäureester, bezogen auf die eingesetzte Menge an Alkanol, mindert. Setzt man die im Beisein der niedermolekularen Aldehyde gebildeten Acrylsäureester in radikalischen Polymerisationen ein, wirkt sich deren Gehalt an den niedermolekularen Aldehyden in der Regel z. B. insofern nachteilig aus, als sie z. B. die Induktionszeit von Polymerisationsreaktionen, d. h., den Zeitraum zwischen dem Erreichen der Polymerisationstemperatur und dem tatsächlichen Beginn der Polymerisation, beeinflussen. Ferner beeinflussen sie in der Regel den Polymerisationsgrad und können in den Polymerisaten auch Verfärbungen verursachen.

Vorgenannte Nachteile treffen normalerweise auch dann zu, wenn man die Roh-Acrylsäure unmittelbar als Acrylsäurequelle in Polymerisationen anwendet.

Es ist daher Aufgabe der Acrylsäurehersteller, die in der Roh-Acrylsäure enthaltenen Verunreinigungen weitestgehend abzutrennen.

Üblicherweise werden zwei Reinheitsgrade zum käuflichen Erwerb angeboten:
- Reinacrylsäure, mit einem Reinheitsgrad von wenigstens 99 Gew.-%, bezogen auf die Summe aller Bestandteile, häufig sogar mit einem Reinheitsgrad von ≥ 99,5 Gew.-%;
- veresterungsgerechte Acrylsäure, mit einem Reinheitsgrad von wenigstens 98 Gew.-%, bezogen auf die Summe aller Bestandteile, häufig sogar mit einem Reinheitsgrad von ≥ 99 Gew.-%.

Reinacrylsäure wird insbesondere zur Herstellung von Superabsorbern (= Massen zur Aufnahme von Wasser auf der Grundlage von Polyacrylsäure und deren Salzen) verwendet und unterliegt diesbezüglich insbesondere dem Erfordernis möglichst keine Diacrylsäure und möglichst kein Dibenzo-1,4-thiazin enthalten zu dürfen, da beide Bestandteile entweder bei der Superabsorberherstellung (insbesondere Dibenzo-1,4-thiazin stört aufgrund seiner radikalische Polymerisationen extrem inhibierenden Wirkung bei der Herstellung von Superabsorbern empfindlich) oder beim Superabsorbergebrauch (Superabsorber finden insbesondere im Hygienebereich Verwendung (z. B. Babywindeln); ein Gehalt an nicht copolymerisierter Diacrylsäure (polymerisiert weniger ausgeprägt als Acrylsäure) ist in diesem Anwendungssektor nicht tolerabel) unerwünscht sind. Die Lagerstabilisierung von Reinacrylsäure gegen unerwünschte vorzeitige radikalische Polymerisation erfolgt daher üblicherweise mittels Hydrochinonmonomethylether oder -monoethylether oder deren Gemischen. Diese Verbindungen wirken vergleichsweise weniger stark inhibierend, was dem bei der Lagerung vergleichsweise geringen Belastungsprofil angemessen ist.

Die Herstellung von Reinacrylsäure erfolgt deshalb normalerweise durch unmittelbare Weiterverarbeitung von frisch hergestellter Roh-Acrylsäure, da selbige praktisch noch keine gebildeten Acrylsäure-Oligomeren enthält. Erzeugte Reinacrylsäure wird üblicherweise frisch verbraucht.

Ferner wird als Reinigungsverfahren zweckmäßigerweise die fraktionierte Kristallisation angewendet, wie sie z. B. in der EP-A 616 998 beschrieben ist, da die fraktionierte Kristallisation bei niedrigen Temperaturen (gemäß Louis F. Fieser und Mary Fieser, Organische Chemie, Verlag Chemie (1975), S. 422, Tabelle 11.1., beträgt der Schmelzpunkt von reiner Acrylsäure bei 1 bar 13°C; durch das Beisein von Fremdkomponenten wird diese Lage der Kristallisationstemperatur noch gesenkt) durchgeführt wird und niedere Temperaturen sowohl die Michael-Addition als auch die radikalische Polymerisation von Acrylsäure zusätzlich hemmen.

Nachteilig an der Reinigung von Roh-Acrylsäure durch Kristallisation ist jedoch die geringe Löslichkeit von Dibenzo-1,4-thiazin in Acrylsäure, die bei 25°C etwa 1,5 Gew.-% und bei 15°C nur noch etwa 0,9 Gew.-%, bezogen auf die aus Acrylsäure und Dibenzo-1,4-thiazin bestehende Lösung, beträgt. Ein Beisein von Essigsäure und/oder Propionsäure in der Acrylsäure beeinflußt diese Löslichkeitswerte kaum, wohingegen ein Beisein von Wasser die Löslichkeit von Dibenzo-1,4-thiazin noch verringert. Dies hat im wesentlichen folgendes zur Konsequenz.

Geht man von einer Roh-Acrylsäure aus, deren Gehalt an Dibenzo-1,4-thiazin oberhalb des Gehaltes des eutektischen Gemisches mit Acrylsäure liegt und kühlt diese Roh-Acrylsäure ab, so fällt als erstes Dibenzo-1,4-thiazin und nicht Acrylsäure aus. D. h., reine Acrylsäure kann nicht abgeschieden werden. Geht man von einer Roh-Acrylsäure aus, deren Gehalt an Dibenzo-1,4-thiazion unterhalb des Gehaltes des eutektischen Gemisches mit Acrylsäure liegt und kühlt diese Roh-Acrylsäure ab, so fällt zwar beim Abkühlen als erstes reine Acrylsäure aus und kann als solche abgeschieden werden. Mit einer solchen Abscheidung von reiner Acrylsäure geht jedoch eine Anreicherung des Dibenzo-1,4-thiazin in der verbleibenden Schmelze einher, bis die eutektische Zusammensetzung erreicht ist und sich nicht mehr reine Acrylsäure sondern das eutektische Gemisch aus Dibenzo-1,4-thiazin und Acrylsäure abscheidet.

Je geringer die Löslichkeit eines Bestandteils in Acrylsäure bei einer bestimmten Temperatur ist, desto geringer ist normalerweise dessen Gewichtsanteil am eutektischen Gemisch. Ist nun aber bereits bei einem sehr geringen Gehalt an Dibenzo-1,4-thiazin die eutektischte Zusammensetzung erreicht, so ist mittels fraktionierter Kristallisation keine weitere Abscheidung reiner Acrylsäure möglich. Vielmehr stellt das eutektische Gemisch nicht verkäuflichen Abfall dar. Allenfalls durch Hilfsmaßnahmen wie Aufschmelzen des eutektischen Gemisches und anschließendes selektives Fällen des enthaltenen Debenzo-1,4-thiazin durch Zusatz von selektiven Fällungsmitteln wie z.B. Wasser oder durch die Anwendung von besonderen Kristallisationverfahren, bei denen die Kristallisationsflächen z.B. mit Impfkristallen eines der Bestandteile des eutektischen Gemisches bestückt werden, konnte hier bisher abgeholfen werden.

Die Aufgabe der vorliegenden Erfindung bestand daher darin, mittels einer geeigneten Maßnahme die Löslichkeit von Dibenzo-1,4-thiazin in Acrylsäure zu erhöhen, dadurch den Anteil von Dibenzo-1,4-thiazin am entsprechenden eutektischen Gemisch zu erhöhen und so ein verbessertes Verfahren zur Reinigung von Roh-Acrylsäure durch Kristallisation zur Verfügung zu stellen.

Überraschenderweise wurde nun gefunden, daß mit zunehmendem Gehalt der Acrylsäure an Diacrylsäure (Michael-Addukt der Acrylsäure an sich selbst) die Löslichkeit von Dibenzo-1,4-thiazin in Acrylsäure insbesondere bei den kristallisationsrelevanten Temperaturen um mehr als 100 % erhöht werden kann.

Erfindungsgemäß wird somit ein Verfahren zur Reinigung von Roh-Acrylsäure durch Kristallisation zur Verfügung gestellt, das dadurch gekennzeichnet ist, daß man den Gehalt der Roh-Acrylsäure an Diacrylsäure vorab des Kristallisationsschrittes auf einen Wert von wenigstens 1 Gew.-%, bezogen auf das Gewicht der Roh-Acrylsäure, einstellt.

Erfindungsgemäße Verfahren sind demnach solche Verfahren zur Reinigung von Roh-Acrylsäure, deren Gehalt an Diacrylsäure vorab des Kristallisationsschrittes auf einen wie vorstehend bezogenen Wert von 1 bis 2 Gew.-%, oder 1 bis 3 Gew.-%, oder 1 bis 5 Gew.-%, oder 1 bis 10 Gew.-%, oder 1 bis 20 Gew.-%, oder 1 bis 40 Gew.-% eingestellt wird. Ein oberhalb von 50 Gew.-% liegender Diacrylsäuregehalt der kristallisativ zu reinigenden Roh-Acrylsäure ist erfindungsgemäß in der Regel nicht zweckmäßig.

Selbstverständliche ist das erfindungsgemäße Verfahren somit anwendbar auf Roh-Acrylsäure, deren Gehalt an Dibenzo-1,4-thiazin, bezogen auf das Gewicht der Roh-Acrylsäure abzüglich der darin enthaltenen Menge an Acrylsäure-Oligomeren, ≥ 0,9 Gew.-%, oder ≥ 1 Gew.-%, oder ≥ 1,1 Gew.-%, oder ≥ 1,2 Gew.-%, oder ≥ 1,3 Gew.-%, oder ≥ 1,4 Gew.-%, oder ≥ 1,5 Gew.-% beträgt. Normalerweise wird der so bezogene Gehalt an Dibenzo-1,4-thiazin nicht mehr als 2 Gew.-% betragen. Das erfindungsgemäße Verfahren ist auch dann anwendbar, wenn die Roh-Acrylsäure in Mengen von bis zu 5 Gew.-% Hydrochinon enthält.

Vorteilhaft für das erfindungsgemäße Verfahren ist, daß Diacrylsäure in Acrylsäure selbst eine erhöhte Löslichkeit aufweist. Ferner ist es vorteilhaft, daß Diacrylsäure unter Anwendung erhöhter Temperatur in Acrylsäure rückgespalten werden kann.

Die erfindungsgemäß erforderliche Einstellung des Diacrylsäuregehaltes der kristallisativ zu reinigenden Roh-Acrylsäure kann auf unterschiedliche Art und Weise realisiert werden. Zum einen kann der zu reinigenden Roh-Acrylsäure eine geringe Menge einer starken protischen Mineralsäure wie z. B. H₂SO₄, HCl oder H₃PO₄ zugesetzt werden, die die Michael-Addition der Acrylsäure katalysiert. Beisein von H₂O begünstigt ebenfalls die Diacrylsäurebildung. Nach ausreichender Diacrylsäurebildung, die gegebenenfalls mittels mäßiger Temperaturerhöhung zusätzlich unterstützt werden kann, kann die katalytische Wirkung der Mineralsäure durch Zusatz einer neutralisierenden Base wieder aufgehoben werden. Selbstverständlich kann der Gehalt an Diacrylsäure auch dadurch erhöht werden, daß man in ersten Trennschritten einen Teil der übrigen enthaltenen Bestandteile abtrennt, z. B. durch Destillation und/ oder kristallisativ.

Alternativ zu den vorgenannten Maßnahmen kann man aber auch in reiner Acrylsäure Diacrylsäure züchten, sie z. B. auf destillativem Weg abtrennen und der kristallisativ zu reinigenden Roh-Acrylsäure zufügen.

Selbstredend wird das erfindungsgemäße Verfahren normalerweise bei Normaldruck, d. h., bei einem Druck von 1 bar durchgeführt.

Üblicherweise wird das erfindungsgemäße Verfahren als fraktionierte Kristallisation verwirklicht. Die einzelnen Kristallisationsstufen können sowohl als statische und/oder als dynamische Kristallisation verwirklicht werden. Sowohl Schichtkristallisation wie Fallfilmkristallisation als auch Suspensionskristallisation können angewendet werden. Eine ausführliche Beschreibung dieser Kristallisationsweisen findet sich in der EP-A 616 998 sowie in der darin zitierten Literatur.

Von den Vorteilen des erfindungsgemäßen Verfahrens kann man im Rahmen einer geschickt gekoppelten Herstellung von veresterungsgerechter Acrylsäure und Reinacrylsäure nicht nur dann Gebrauch machen, wenn von einer Roh-Acrylsäure gemäß der Definition in vorliegender Schrift ausgegangen wird, sondern auch dann, wenn von einer rohen Acrylsäure ausgegangen wird, die eine von 0 verschiedene, aber unterhalb der erfindungsgemäßen Grenze von 0,8 Gew.-% liegende, Menge an Dibenzo-1,4-thiazin enthält.

Dabei wird in ersten Kristallisations- und/oder Destillationsschritten zunächst kontinuierlich veresterungsgerechte Acrylsäure erzeugt. Da selbige im Rahmen der Veresterung prozeßstabilisiert werden muß, wirkt sich in selbiger noch enthaltenes Dibenzo-1,4-thiazin nicht nachteilig aus (für veresterungsgerechte Acrylsäure liegt die einzuhaltende Obergrenze des Gehaltes an Dibenzo-1,4-thiazin bei 750 ppm (auf das Gewicht bezogen) und die entsprechende Grenze für Diacrylsäure liegt bei 1,5 Gew.-%; für Reinacrylsäure liegt die einzuhaltende Obergrenze des Gehaltes an Dibenzo-1,4-thiazin bei 1 ppm und die entsprechende Grenze für Diacrylsäure liegt bei 0,2 Gew.-% im Fall einer Verwendung für übliche Polymerisationen und bei 500 ppm im Fall einer Verwendung zur Herstellung von Superabsorbern;).

Die erzeugte veresterungsgerechte, Dibenzo-1,4-thiazin enthaltende, Acrylsäure wird dann wenigstens teilweise zwischengelagert, wobei diese Zwischenlagerung wenige Tage, eine Woche, einen Monat, ein Vierteljahr, ein halbes Jahr oder länger dauern kann.

In diesem Zwischenlager enthaltene Acrylsäure kann dann je nach Bedarf zur Herstellung von Acrylsäureestern oder zur kristallisativen Herstellung von Reinacrylsäure eingesetzt werden. Während der Lagerung zwischenzeitlich erfolgende Bildung von Diacrylsäure, die üblicherweise im Rahmen der Reinacrylsäureherstellung zu verhindern versucht wird, macht sich bei der Veresterung nur begrenzt nachteilig bemerkbar, da via einer im Rahmen der Aufarbeitung der Veresterungsgemisches einbezogenen Rückspaltung letztlich, auf eingesetztes Alkanol bezogene, Ausbeuteverluste verhindert werden können. Wie im Rahmen dieser Schrift ausgewiesen wird, wirkt sich die im Verlauf der Lagerung gebildete Diacrylsäure jedoch vorteilhaft auf die kristallisative Weiterreinigung von veresterungsgerechter Acrylsäure zur Herstellung von an Dibenzo-1,4-thiazin im wesentlichen freier Reinacrylsäure aus.

Die nachfolgende Tabelle 1 weist die mit zunehmendem Diacrylsäuregehalt einhergehende Löslichkeitszunahme von Dibenzo-1,4-thiazin in Acrylsäure für zwei angewendete Temperaturen als Ergebnis von Löslichkeitsversuchen aus.

**Tabelle 1**

| | Löslichkeit von Dibenzo-1,4-thiazin (in Gew.-%, bezogen auf Säure) | |
|---|---|---|
| Zusammensetzung der Säure | 15°C | 25°C |
| 100 Gew.-% Acrylsäure | 0,9 Gew.-% | 1,5 Gew.-% |
| | | |
| 97 Gew.-% Acrylsäure 3 Gew.-% Diacrylsäure | 1,45 Gew.-% | 2,10 Gew.-% |
| | | |
| 95 Gew.-% Acrylsäure 5 Gew.-% Diacrylsäure | 1,71 Gew.-% | 2,15 Gew.-% |
| | | |
| 90 Gew.-% Acrylsäure 10 Gew.-% Diacrylsäure | 1,83 Gew.-% | 2,25 Gew.-% |
| | | |
| 80 Gew.-% Acrylsäure 20 Gew.-% Diacrylsäure | 2,01 Gew.-% | 2,51 Gew.-% |
| | | |
| 73 Gew.-% Acrylsäure 27 Gew.-% Diacrylsäure | | 2,60 Gew.-% |
| | | |
| 55 Gew.-% Acrylsäure 45 Gew.-% Diacrylsäure | | 2,90 Gew.-% |
| | | |
| 10 Gew.-% Acrylsäure 90 Gew.-% Diacrylsäure | | 3,20 Gew.-% |

Die nachfolgende Tabelle 2 weist die Löslichkeit von Dibenzo-1,4,-thiazin in Essigsäure aus.

**Tabelle 2**

| Temperatur (°C) | Löslichkeit (Gew.-%, bezogen auf Lösung) |
|---|---|
| 5 | 0,28 |
| 10 | 0,89 |
| 20 | 1,25 |
| 30 | 1,53 |

## Patentansprüche

1. Verfahren zur Reinigung von Roh-Acrylsäure, die, läßt man ihren Gehalt an Acrylsäure-Oligomeren (Michael-Addukte) unberücksichtigt,
≥ 70 Gew.-% Acrylsäure,
≤ 20 Gew.-% Essigsäure,
≤ 5 Gew.-% Propionsäure,
≤ 5 Gew.-% Wasser,
≤ 5 Gew.-% niedermolekulare Aldehyde und
≥ 0,80 Gew.-% Dibenzo-1,4-thiazin
enthält, durch Kristallisation, dadurch gekennzeichnet, daß man den Gehalt der Roh-Acrylsäure an Diacrylsäure (Michael-Addukt) vorab des Kristallisationsschrittes auf einen Wert von wenigstens 1 Gew.-%, bezogen auf das Gesamtgewicht (d. h. einschließlich der Acrylsäure-Oligomere) der Roh-Acrylsäure, einstellt.

2. Verfahren nach Anspruch 1, dadurch gekennzeichnet, daß man den Gehalt der Roh-Acrylsäure an Diacrylsäure vorab des Kristallisationsschrittes auf einen Wert von wenigstens 5 Gew.-%, bezogen auf das Gesamtgewicht der Roh-Acrylsäure, einstellt.

3. Verfahren nach Anspruch 1 oder 2, dadurch gekennzeichnet, daß die kristallisativ zu reinigende Roh-Acrylsäure, läßt man ihren Gehalt an Acrylsäure-Oligomeren unberücksichtigt, ≥ 0,90 Gew.-% Dibenzo-1,4-thiazin enthält.

4. Verfahren nach Anspruch 1 oder 2, dadurch gekennzeichnet, daß die kristallisativ zu reinigende Roh-Acrylsäure, läßt man ihren Gehalt an Acrylsäure-Oligomeren unberücksichtigt, ≥ 1,0 Gew.-% Dibenzo-1,4-thiazin enthält.

5. Verfahren zur Herstellung von veresterungsgerechter Acrylsäure sowie von Reinacrylsäure durch kristallisative Reinigung von roher, Dibenzo-1,4-thiazin enthaltender, Acrylsäure, dadurch gekennzeichnet, daß man aus der rohen Acrylsäure zunächst durch fraktionierte Kristallisation und/oder Destillation veresterungsgerechte Acrylsäure erzeugt, die veresterungsgerechte Acrylsäure wenigstens teilweise zwischengelagert und bei Bedarf aus diesem Zwischenlager einen erhöhten Diacrylsäuregehalt aufweisende Acrylsäure entnimmt und anschließend gemäß Anspruch 1 kristallisativ zu Reinacrylsäure weiterreinigt.

## Claims

1. A process for purifying by crystallization a crude acrylic acid comprising, if its acrylic acid oligomer (Michael adduct) content is ignored,
≥ 70 % by weight of acrylic acid,
≤ 20 % by weight of acetic acid,
≤ 5 % by weight of propionic acid,
≤ 5 % by weight of water,
≤ 5 % by weight of low molecular weight aldehydes, and
≥ 0.80 % by weight of 1,4-dibenzothiazine,
which comprises adjusting the diacrylic acid (Michael adduct) content of the crude acrylic acid to a value of at least 1% by weight, based on the total weight (i.e. including the acrylic acid oligomers) of the crude acrylic acid, prior to the crystallization step.

2. A process as claimed in claim 1, wherein the diacrylic acid content of the crude acrylic acid is adjusted to a value of at least 5% by weight, based on the total weight of the crude acrylic acid, prior to the crystallization step.

3. A process as claimed in claim 1 or 2, wherein the crude acrylic acid to be purified by crystallization comprises ≥ 0.90% by weight of 1,4-dibenzothiazine, if its acrylic acid oligomer content is ignored.

4. A process as claimed in claim 1 or 2, wherein the crude acrylic acid to be purified by crystallization comprises ≥ 1.0% by weight of 1,4-dibenzothiazine, if its acrylic acid oligomer content is ignored.

5. A process for preparing esterification-grade acrylic acid and glacial acrylic acid by crystallizational purification of a crude acrylic acid comprising 1,4-dibenzothiazine, which comprises initially subjecting the crude acrylic acid to a fractional crystallization and/or distillation to generate esterification-grade acrylic acid, intermediately storing at least some of the esterification-grade acrylic acid, and withdrawing acrylic acid having an increased diacrylic acid content from this intermediate store as required and subsequently further purifying it by crystallization as claimed in claim 1 to generate glacial acrylic acid.

## Revendications

1. Procédé de purification d'acide acrylique brut, contenant, si on ne tient pas compte de sa teneur en oligomères d'acide acrylique (adduits de Michael),
≥ 70 % en poids d'acide acrylique,
≤ 20 % en poids d'acide acétique,
≤ 5 % en poids d'acide propionique,
≤ 5 % en poids d'eau,
≤ 5 % en poids d'aldéhydes de bas poids moléculaire, et
≥ 0,80 % en poids de dibenzo-1,4-thiazine,
par cristallisation, caractérisé en ce que l'on règle la teneur de l'acide acrylique brut en acide diacrylique (adduits de Michael) avant l'étape de cristallisation, à une valeur d'au moins 1 % en poids, par rapport au poids total (c'est à dire y compris les oligomères d'acide acrylique) de l'acide acrylique brut.

2. Procédé selon la revendication 1, caractérisé en ce que l'on règle la teneur de l'acide acrylique brut en acide diacrylique, avant l'étape de cristallisation, à une valeur d'au moins 5% en poids, par rapport au poids total de l'acide acrylique brut.

3. Procédé selon la revendication 1 ou 2, caractérisé en ce que l'acide acrylique brut à purifier par cristallisation contient ≥ 0,90% en poids de dibenzo-1,4-thiazine, si on ne tient pas compte de sa teneur en oligomères d'acide acrylique brut.

4. Procédé selon la revendication 1 ou 2, caractérisé en ce que l'acide acrylique brut à purifier par cristallisation contient ≥ 1,00% en poids de dibenzo-1,4-thiazine, si on ne tient pas compte de sa teneur en oligomères d'acide acrylique.

5. Procédé de préparation d'acide acrylique rectifié pour estérification et d'acide acrylique pur, par purification par cristallisation d'acide acrylique brut contenant de la dibenzo-1,4-thiazine, caractérisé en ce que l'on produit tout d'abord, à partir de l'acide acrylique brut, de l'acide acrylique rectifié pour estérification par cristallisation fractionnée et/ou distillation, on entrepose de façon intermédiaire au moins une partie de l'acide acrylique rectifié pour estérification, et, au besoin, on extrait de cet entreposage intermédiaire un acide acrylique comportant une teneur en acide diacrylique plus élevée, et ensuite on le purifie par cristallisation selon la revendication 1, pour donner de l'acide acrylique pur.
